# EUROPEAN PATENT SPECIFICATION

(11) **EP 4 450 027 B1**
(45) Date of publication and mention of the grant of the patent: **23.09.2026**
(21) Application number: 24163291.8
(22) Date of filing: 13.03.2024
(51) Int. Cl.: A61F 2/24

(54) **PROSTHETIC HEART VALVE IMPLANT APPARATUS**
PROTHETISCHE HERZKLAPPENIMPLANTATVORRICHTUNG
APPAREIL D'IMPLANT DE VALVULE CARDIAQUE PROTHÉTIQUE

(30) Priority: 21.04.2023 US 202363460963 P; 21.02.2024 US 202418582929
(43) Date of publication of application: 23.10.2024
(73) Proprietor: Medtronic, Inc., Minneapolis, Minnesota 55432 (US)
(72) Inventor: Menon, Grant Alexander, Minneapolis (US)
(74) Representative: Zimmermann & Partner Patentanwälte mbB

(56) References cited:
- WO-A1-2006/026912

## Description

### CROSS-REFERENCE TO RELATED APPLICATIONS

This application claims the benefit of U.S. Provisional Application No. 63/460,963, filed April 21, 2023.

### FIELD

The present disclosure relates generally to a transcatheter heart valve implant apparatus and, more particularly, to a transcatheter heart valve implant apparatus configured to resist unintended movement during deployment of a heart valve prosthesis, thereby enabling controlled positioning of the heart valve prosthesis within a patient.

### BACKGROUND

It is known to provide a heart valve implant apparatus for implanting a heart valve prosthesis within a target site of the vasculature of a patient. The heart valve prosthesis can expand from a radially-collapsed position to a radially-expanded position. However, delivery of the heart valve prosthesis can be difficult because expansion of the heart valve prosthesis during deployment can cause unintended movement, resulting in less-controlled positioning within a patient.
WO 2006/026912 A1 relates to an implantable artificial heart valve.

### SUMMARY

The following presents a simplified summary of the disclosure to provide a basic understanding of some aspects described in the detailed description. The invention relates to a transcatheter heart valve implant assembly as defined in claim 1. Embodiments of the invention are recited in the dependent claims.

In aspects, a transcatheter heart valve implant apparatus comprises an annular valve prosthesis extending along a longitudinal axis between an inflow end of the valve prosthesis and an outflow end of the valve prosthesis. The transcatheter heart valve implant apparatus comprises an inner shaft member extending along a shaft axis through the valve prosthesis between a proximal shaft end and a distal shaft end. The inner shaft member comprises a first lumen extending between the proximal shaft end and a first exit opening at the distal shaft end. The inner shaft member further comprises a second lumen extending adjacent to the first lumen between the proximal shaft end and a second exit opening spaced a distance from the distal shaft end. A shaft wall is positioned between the first lumen and the second lumen. The transcatheter heart valve implant apparatus comprises a first guidewire extending through the first lumen and the first exit opening. A first end of the first guidewire is configured to be positioned at a first anchoring location. The transcatheter heart valve implant apparatus comprises a second guidewire extending through the second lumen and the second exit opening. A second end of the second guidewire is configured to be positioned at a second anchoring location simultaneously with the first guidewire positioned at the first anchoring location.

In aspects, the transcatheter heart valve implant apparatus comprises a tip attached to the distal shaft end. The tip comprises a tip lumen that is aligned with the first lumen such that the first guidewire extends through the tip lumen.

In aspects, the second exit opening is spaced apart from the tip.

In aspects, the first anchoring location comprises a first pulmonary artery branch and the second anchoring location comprises a second pulmonary artery branch.

In aspects, the transcatheter heart valve implant apparatus comprises an outer sheath circumferentially surrounding the valve prosthesis and the inner shaft member. The outer sheath is movable relative to the inner shaft member such that in a first delivery position, the second exit opening is within a sheath chamber of the outer sheath, and in a second delivery position, the second exit opening is outside of the sheath chamber.

In aspects, the inner shaft member is configured to be rotatable relative to the outer sheath such that in a first rotational position, the second exit opening faces a first location of the outer sheath, and in a second rotational position, the second exit opening faces a second location of the outer sheath.

In aspects, the first lumen comprises a first diameter that is within a range from about 0.5 millimeters to about 1.5 millimeters, and the second lumen comprises a second diameter that is within a range from about 0.5 millimeters to about 1.5 millimeters.

In aspects, the first lumen is substantially parallel to the second lumen.

In aspects, a transcatheter heart valve implant apparatus comprises an annular valve prosthesis extending along a longitudinal axis between an inflow end of the valve prosthesis and an outflow end of the valve prosthesis. The transcatheter heart valve implant apparatus comprises an inner shaft member extending along a shaft axis through the valve prosthesis between a proximal shaft end and a distal shaft end. The inner shaft member comprises a first lumen extending between a first entry opening at the proximal shaft end and a first exit opening at the distal shaft end. The inner shaft member comprises a second lumen extending adjacent to the first lumen between a second entry opening at the proximal shaft end and a second exit opening spaced a distance from the distal shaft end. A shaft wall is positioned between the first lumen and the second lumen. The transcatheter heart valve implant apparatus comprises a first guidewire extending through the first entry opening, the first lumen, and the first exit opening. A first end of the first guidewire is configured to be positioned in a first pulmonary artery branch. The transcatheter heart valve implant apparatus comprises a second guidewire extending through the second entry opening, the second lumen, and the second exit opening. A second end of the second guidewire is configured to be positioned in a second pulmonary artery branch simultaneously with the first guidewire positioned in the first pulmonary artery branch.

In aspects, the transcatheter heart valve implant apparatus comprises a guide member attached to the proximal shaft end of the inner shaft member.

In aspects, the guide member comprises a first passageway that is aligned with the first entry opening of the first lumen such that the first guidewire extends through the first passageway, the first entry opening, and the first lumen. The first passageway extends along a first guide axis. The guide member comprises a second passageway that is aligned with the second entry opening of the second lumen such that the second guidewire extends through the second passageway, the second entry opening, and the second lumen. The second passageway extends along a second guide axis.

In aspects, an angle between the first guide axis and the second guide axis is within a range from about 20 degrees to about 60 degrees.

In aspects, the transcatheter heart valve implant apparatus comprises a tip attached to the distal shaft end. The tip comprises a tip lumen that is aligned with the first lumen such that the first guidewire extends through the tip lumen.

In aspects, the second exit opening is spaced apart from the tip.

In aspects, methods of implanting a valve prosthesis comprise positioning a first guidewire within a first lumen of an inner shaft member. The first lumen extends between a proximal shaft end of the inner shaft member and a distal shaft end of the inner shaft member. Methods comprise positioning a second guidewire within a second lumen of the inner shaft member. The second lumen extends between the proximal shaft end and a second exit opening spaced a distance from the distal shaft end. Methods comprise guiding the first guidewire through a first exit opening at the distal shaft end such that a first guidewire end of the first guidewire is positioned in a first anchoring location. Methods comprise moving a transcatheter heart valve implant apparatus comprising the inner shaft member and the valve prosthesis along the first guidewire toward a treatment site within a body lumen. Methods comprise guiding the second guidewire through the second exit opening such that a second guidewire end of the second guidewire is positioned in a second anchoring location. Methods comprise deploying the valve prosthesis at the treatment site while the first guidewire end is positioned in the first anchoring location and the second guidewire end is positioned in the second anchoring location.

In aspects, during the moving of the transcatheter heart valve implant apparatus, an outer sheath circumferentially surrounds the valve prosthesis and the inner shaft member, and the second exit opening is within a sheath chamber of the outer sheath.

In aspects, prior to guiding the second guidewire through the second exit opening, methods comprise moving one or more of the outer sheath relative to the inner shaft member or the inner shaft member relative to the outer sheath such that the second exit opening is outside the sheath chamber.

In aspects, prior to guiding the second guidewire through the second exit opening, methods comprise rotating the inner shaft member relative to the outer sheath such that the second exit opening is oriented facing the second anchoring location.

In aspects, the rotating occurs while the second exit opening is outside the sheath chamber.

In aspects, during the moving of the transcatheter heart valve implant apparatus, the second guidewire end is located within the second lumen. The first anchoring location comprises a first pulmonary artery branch and the second anchoring location comprises a second pulmonary artery branch.

Additional features and advantages of the aspects disclosed herein will be set forth in the detailed description that follows, and in part will be clear to those skilled in the art from that description or recognized by practicing the aspects described herein, including the detailed description which follows, the claims, as well as the appended drawings. It is to be understood that both the foregoing general description and the following detailed description present aspects intended to provide an overview or framework for understanding the nature and character of the aspects disclosed herein. The accompanying drawings are included to provide further understanding and are incorporated into and constitute a part of this specification. The drawings illustrate various aspects of the disclosure, and together with the description explain the principles and operations thereof.

### BRIEF DESCRIPTION OF THE DRAWINGS

These and other features, aspects and advantages are better understood when the following detailed description is read with reference to the accompanying drawings, in which:
**FIG. 1** schematically illustrates example aspects of a transcatheter heart valve implant apparatus in accordance with aspects of the disclosure;
**FIG. 2** schematically illustrates a distal end of the transcatheter heart valve implant apparatus in accordance with aspects of the disclosure;
**FIG. 3** illustrates deployment of a heart valve prosthesis in accordance with aspects of the disclosure;
**FIG. 4** illustrates deployment of the heart valve prosthesis in accordance with aspects of the disclosure;
**FIG. 5** illustrates a cross-sectional view of the distal end of the transcatheter heart valve implant apparatus along lines **5-5** of **FIG. 2** in accordance with aspects of the disclosure;
**FIG. 6** illustrates a first guidewire extending through an inner shaft member of the transcatheter heart valve implant apparatus in accordance with aspects of the disclosure;
**FIG. 7** illustrates a second guidewire extending through the inner shaft member of the transcatheter heart valve implant apparatus in accordance with aspects of the disclosure;
**FIG. 8** illustrates the second guidewire extending from the inner shaft member in accordance with aspects of the disclosure;
**FIG. 9** illustrates the first guidewire and the second guidewire simultaneously extending from the inner shaft member during deployment of the heart valve prosthesis in accordance with aspects of the disclosure;
**FIG. 10** illustrates a side view of the transcatheter heart valve implant apparatus with the first guidewire positioned in a right pulmonary artery in accordance with aspects of the disclosure;
**FIG. 11** illustrates a side view of the transcatheter heart valve implant apparatus with the inner shaft member moving relative to an outer sheath in accordance with aspects of the disclosure;
**FIG. 12** illustrates a side view of the inner shaft member rotating relative to the outer sheath in accordance with aspects of the disclosure;
**FIG. 13** illustrates a side view of the transcatheter heart valve implant apparatus with the second guidewire positioned in a left pulmonary artery in accordance with aspects of the disclosure; and
**FIG. 14** illustrates the first guidewire and the second guidewire simultaneously positioned in pulmonary arteries during deployment of the heart valve prosthesis in accordance with aspects of the disclosure.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

As used herein, the term "about" means that amounts, sizes, formulations, parameters, and other quantities and characteristics are not, and need not be, exact, but may be approximate and/or larger or smaller, as desired, reflecting tolerances, conversion factors, rounding off, measurement error and the like, and other factors known to those of skill in the art.

Ranges can be expressed herein as from "about" one value, and/or to "about" another value. When such a range is expressed, aspects include from the one value to the other value. Similarly, when values are expressed as approximations by use of the antecedent "about," it will be understood that the value forms another aspect. It will be further understood that the endpoints of each of the ranges are significant both in relation to the other endpoint, and independently of the other endpoint.

Directional terms as used herein - for example up, down, right, left, front, back, top, bottom, upper, lower, etc. - are made only with reference to the figures as drawn and are not intended to imply absolute orientation.

Unless otherwise expressly stated, it is in no way intended that any methods set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred in any respect. This holds for any possible non-express basis for interpretation, including matters of logic relative to arrangement of steps, operational flow, order of components, or orientation of components; plain meaning derived from grammatical organization or punctuation, and; the number or type of aspects described in the specification.

As used herein, the singular forms "a," "an," and "the" include plural references unless the context clearly dictates otherwise. Thus, for example, reference to "a" component includes aspects having two or more such components, unless the context clearly indicates otherwise.

The word "exemplary," "example," or various forms thereof are used herein to mean serving as an example, instance, or illustration. Any aspect or design described herein as "exemplary" or as an "example" should not be construed as preferred or advantageous over other aspects or designs. Furthermore, examples are provided solely for purposes of clarity and understanding and are not meant to limit or restrict the disclosed subject matter or relevant portions of this disclosure in any manner. It can be appreciated that a myriad of additional or alternate examples of varying scope could have been presented but have been omitted for purposes of brevity.

As used herein, the terms "comprising," "including," and variations thereof shall be construed as synonymous and open-ended, unless otherwise indicated. A list of elements following the transitional phrases comprising or including is a non-exclusive list, such that elements in addition to those specifically recited in the list may also be present.

The terms "substantial," "substantially," and variations thereof as used herein are intended to represent that a described feature is equal or approximately equal to a value or description. For example, a "substantially planar" surface is intended to denote a surface that is planar or approximately planar. Moreover, "substantially" is intended to denote that two values are equal or approximately equal. The term "substantially" may denote values within about 10% of each other, for example, within about 5% of each other, or within about 2% of each other.

Modifications may be made to the instant disclosure without departing from the scope or spirit of the claimed subject matter. Unless specified otherwise, "first," "second," or the like are not intended to imply a temporal aspect, a spatial aspect, an ordering, etc. Rather, such terms are merely used as identifiers, names, etc. for features, elements, items, etc. For example, a first end and a second end generally correspond to end A and end B or two different ends.

Unless otherwise indicated, the terms "distal" and "proximal" are used in the following description with respect to a position or direction relative to the treating clinician. "Distal" and "distally" are positions distant from or in a direction away from the clinician, and "proximal" and "proximally" are positions near or in a direction toward the clinician. In addition, the term "self-expanding" may be used in the following description with reference to one or more valve or stent structures of the prostheses hereof and is intended to convey that the structures are shaped or formed from a material that can be provided with a mechanical memory to return the structure from a compressed or constricted delivery configuration to an expanded deployed configuration or vice versa. Non-exhaustive exemplary self-expanding materials include stainless steel, a pseudoelastic metal such as a nickel titanium alloy or nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in aspects hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

Diseases associated with heart valves, such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses generally include a frame or stent and a prosthetic valve mounted within the frame. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

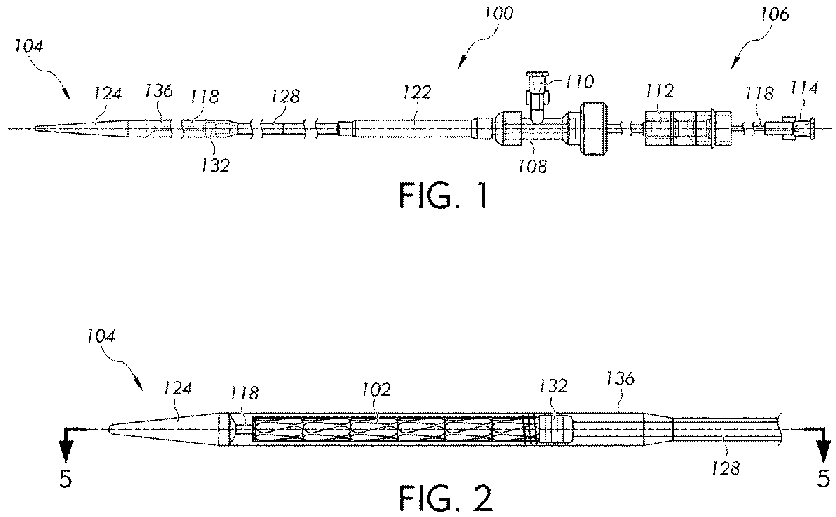

**FIGS. 1-14** illustrate an example transcatheter heart valve implant apparatus **100** for delivering a heart valve prosthesis **102.** The transcatheter heart valve implant apparatus **100** is illustrated to facilitate description of the disclosure. The following description of the transcatheter heart valve implant apparatus **100** and the heart valve prosthesis **102** is merely exemplary in nature and is not intended to limit the scope of the application.

**FIG. 1** illustrates the transcatheter heart valve implant apparatus **100** having and extending between a distal end **104** and a proximal end **106.** The distal end **104** can be used to load and deliver the heart valve prosthesis **102** (e.g., illustrated in **FIG. 2**). The proximal end **106** can comprise components, for example, those found in other catheter delivery systems for controlling transcatheter delivery of the heart valve prosthesis **102** through the vasculature of the patient and for controlling deployment of the heart valve prosthesis **102** at the target site. In aspects, the components at the proximal end **106** may comprise, for example, a first rotating homeostasis valve **108,** a side access port **110,** a second rotating homeostasis valve **112,** and a guide member **114.** The first rotating homeostasis valve **108** can comprise latex and can form a fluid seal to limit blood or other fluid from leaking out of the transcatheter heart valve implant apparatus **100** at the proximal end **106** or entry site into a patient. The side access port **110** is provided to inject contrast media or saline, for example, into the transcatheter heart valve implant apparatus **100.** The second rotating homeostasis valve **112** may be similar to the first rotating homeostasis valve **108** and can limit blood or other fluid from leaking back through the transcatheter heart valve implant apparatus **100.** In addition, the second rotating homeostasis valve **112** can allow wires, devices, and fluid to pass through to aid in the preparation, delivery, and deployment of the heart valve prosthesis **102.** Furthermore, the second rotating homeostasis valve **112** can control the components of the distal end **104.** For example, by manipulating (e.g., rotating) a portion of the second rotating homeostasis valve **112,** a physician can control components of the distal end **104.** The first and second homeostasis valves **108, 112** can be compatible with catheter-laboratory components and, unless otherwise noted, the heart valve implant apparatus **100** is not intended to be limited to the specific components and features disclosed.

In aspects, the transcatheter heart valve implant apparatus **100** can be inserted into a vessel or artery of a patient, for example, the femoral artery. The proximal end **106** can extend outside of the patient, for example, in the groin area, while the distal end **104** may be delivered intravascularly to an area at or near a pulmonary valve inside the body. However, insertion of the transcatheter heart valve implant apparatus **100** in other areas of the body are also contemplated. The transcatheter heart valve implant apparatus **100** can include one or more axial lumens to pass items (e.g., guidewires, valve prosthesis, contrast media, other catheters, etc.) through the transcatheter heart valve implant apparatus **100.**

In aspects, the transcatheter heart valve implant apparatus **100** can comprise an inner shaft member **118** comprising at least one guidewire lumen. As used herein, a lumen comprises a cavity or hollow bore that extends through a structure, for example, the inner shaft member **118.** The guide member **114** can be attached to the inner shaft member **118** at the proximal end **106.** In aspects, the inner shaft member **118** can extend partially or completely along an entire length of the transcatheter heart valve implant apparatus **100** and may be slid or moved through other components of the transcatheter heart valve implant apparatus **100.** As explained herein, a plurality of guidewires can extend through the inner shaft member **118** and can be used to guide the heart valve prosthesis **102** to a desired location. The transcatheter heart valve implant apparatus **100** can comprise an outer shaft **122** that is located between the distal end **104** and the proximal end **106,** with the inner shaft member **118** extending through the outer shaft **122.** The outer shaft **122** can limit blood from leaking around the transcatheter heart valve implant apparatus **100** and provides a relatively smooth, flexible length to enable the heart valve implant apparatus **100** to traverse through the vasculature of the patient.

In aspects, a tapered tip **124** can be attached to the inner shaft member **118** at the distal end **104.** The tip **124** comprises a tapered shape to ease the passage of the implant apparatus **100** into and through the vasculature. In aspects, a holding catheter **128** can be attached to the heart valve prosthesis **102.** The holding catheter **128** can comprise a centrally located lumen surrounding the inner shaft member **118.** The holding catheter **128** can slide relative to the inner shaft member **118** and may be controlled by the second rotating homeostasis valve **112** at the proximal end **106** of the implant apparatus **100.** In aspects, a portion of the second rotating homeostasis valve **112** can be rotated or otherwise manipulated in order to rotate the holding catheter **128** or move the holding catheter **128** proximally and distally as desired. In aspects, a coil **132** can be attached to the distal end of the holding catheter **128.** In this way, movement of the holding catheter **128,** as controlled by the second rotating homeostasis valve **112,** can control movement or rotation of the coil **132.** The coil **132** can be removably attached to the valve prosthesis **102.** As such, the coil **132** can allow for loading of the valve prosthesis **102,** holding of the valve prosthesis **102** during delivery, and release of the valve prosthesis **102** upon reaching a treatment site. In aspects, rotation of the coil **132** can cause the valve prosthesis **102** to detach from the coil **132.** The implant apparatus **100** can further comprise an outer sheath **136** that may comprise a low friction and flexible material, such as polytetrafluoroethylene (PTFE), polyurethane, silicone, or polyethylene. The outer sheath **136** can be sized and shaped to receive components near the distal end **104,** for example, the inner shaft member **118,** the coil **132,** the valve prosthesis **102,** etc.

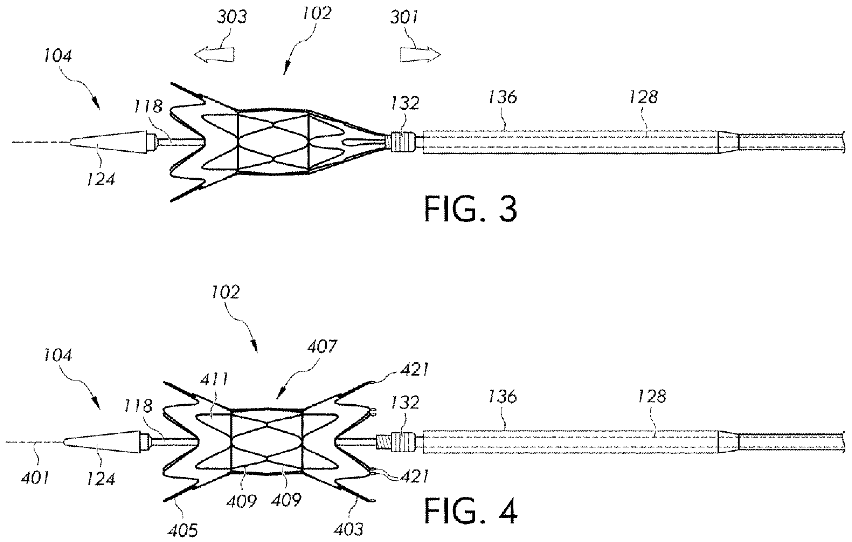

**FIG. 2** illustrates the distal end **104** of the implant apparatus **100.** In aspects, the valve prosthesis **102** can be in a radially-compressed position during intraluminal delivery to a treatment site. In the radially-compressed position, the valve prosthesis **102** can be removably attached to the coil **132** and circumferentially surrounded by the outer sheath **136.** The valve prosthesis **102** can extend coaxially with the inner shaft member **118,** for example, with the inner shaft member **118** extending through a center of the valve prosthesis **102.** The outer sheath **136** comprises a cross-sectional size (e.g., diameter) that is less than a cross-sectional size of the valve prosthesis **102** when the valve prosthesis **102** is in a radially-expanded position (e.g., illustrated in **FIG. 4**). As such, the outer sheath **136,** along with the coil **132,** can maintain the valve prosthesis **102** in the radially-compressed position during delivery.

**FIG. 3** illustrates initial stages of deployment of the valve prosthesis **102** upon reaching the treatment site (e.g., heart valve annulus, areas at or near a pulmonary valve, etc.). To deploy or release the valve prosthesis **102,** the outer sheath **136** can be retracted, for example, by moving the outer sheath **136** relative to the valve prosthesis **102** in a proximal direction **301** away from the distal end **104** and toward the proximal end **106.** In addition, or in the alternative, the holding catheter **128** can move relative to the outer sheath **136** in a distal direction **303** toward the distal end **104** and away from the proximal end **106,** which can likewise cause the coil **132** and the valve prosthesis **102** to move in the distal direction **303.** In this way, the valve prosthesis **102** may no longer be circumferentially surrounded by the outer sheath **136** and may begin to move from the radially-compressed position to the radially-expanded position. As illustrated in **FIG. 3****,** an end of the valve prosthesis **102** may remain attached to the coil **132** while the valve prosthesis **102** is removed from the outer sheath **136** (e.g., due to the movement of the outer sheath **136** in the proximal direction **301** and/or movement of the holding catheter **128** in the distal direction **303**).

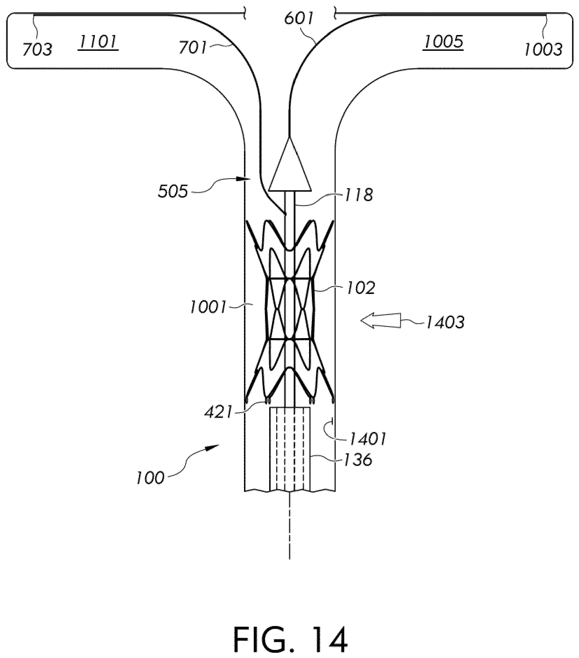

**FIG. 4** illustrates further stages of deployment of the valve prosthesis **102** after the outer sheath **136** has been retracted proximally and the valve prosthesis **102** has been removed from the outer sheath **136** and allowed to self-expand. Once the heart valve prosthesis **102** is removed from the outer sheath **136,** the holding catheter **128** can be remotely rotated from the proximal end **106,** which can cause the coil **132** to likewise rotate. Rotation of the coil **132** can release the valve prosthesis **102** from the coil **132,** for example as shown in **FIG. 3****,** due to attachment members (e.g., loops **421** illustrated in **FIGS. 4** and **14**) at an end of the valve prosthesis **102** being released from the coil **132.** Once the valve prosthesis **102** is released from the coil **132,** the valve prosthesis **102** may expand to the radially-expanded position illustrated in **FIG. 4****.** Portions of the implant apparatus **100,** for example, the inner shaft member, that still extend through a lumen of the valve prosthesis **102** may be withdrawn and retracted, for example, by moving the implant apparatus **100** in the proximal direction **301.**

As illustrated in **FIG. 4****,** the valve prosthesis **102** can extend along a longitudinal axis **401** between an inflow end **403** and an outflow end **405,** wherein the inflow end **403** is attached to the coil **132** in **FIGS. 1-3****.** The valve prosthesis **102** can comprise a stent portion **407** that may move between the radially-expanded position and the radially-compressed position. In this way, the valve prosthesis **102** may be compressible to be inserted via catheter and expandable (e.g., self-expandable) to fit a desired body lumen, such as the right ventricular outflow tract (RVOT), for example. To further facilitate expansion of the valve prosthesis **102,** in aspects, a balloon may be employed to cause the valve prosthesis **102** to move from the radially-compressed position to the radially-expanded position, for example, during a post-implant dilatation procedure. Although disclosed as self-expanding, the valve prosthesis **102** can be one or a combination of self-expandable, balloon-expandable, or mechanically expandable in other embodiments.

The stent portion **407** can comprise one or more materials that may be compressed but may regain a desired configuration upon release from compression. In aspects, the stent portion **407** can comprise a framework comprising a wire or plurality of wires **409** made of a shape-memory material (e.g., NITINOL^{™}) and one or more pieces of fabric **411** (i.e., cloth, material, etc.) to which the wire or wires **409** can be attached. The wires **409** of the stent portion **407** can be shaped and aligned such that when the wires **409** are aligned generally coaxially, a central lumen can run along the length of the stent portion **407.** In aspects, the wires **409** can comprise a series of sinusoidal bends around their circumference, which allow for the compression and expansion of the valve prosthesis **102** with minimal force. The wires **409** may be attached separately to the fabric **411.** In addition, or alternatively, the wires **409** may be attached to each other at some or all of their adjacent apices. The valve prosthesis **102** comprises one or more valve leaflets disposed within and secured to the wires **409,** with the valve leaflets configured to open and close to regulate blood flow through the valve prosthesis **102.** The implant apparatus **100** and the valve prosthesis **102** are further described in U.S. Pat. Nos. 8,801,776 and 9,364,324.

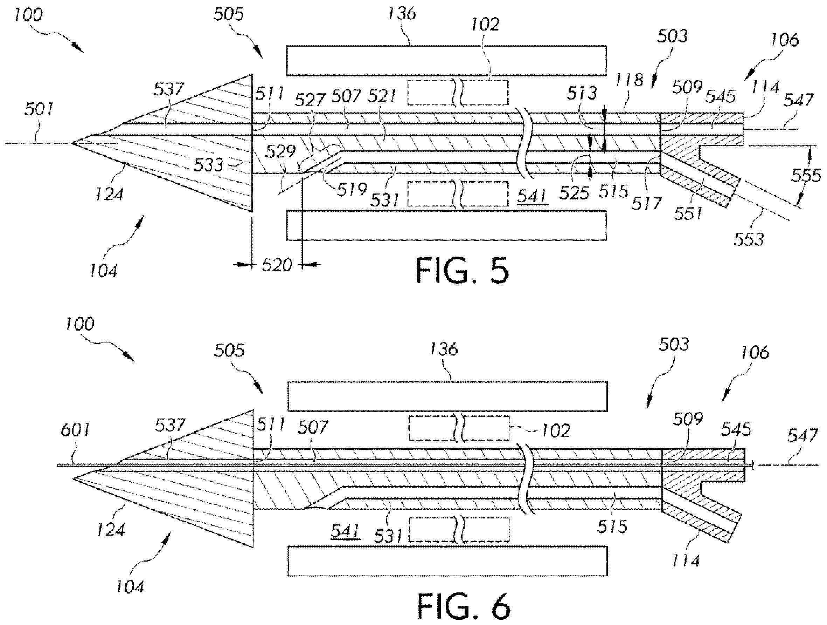

**FIG. 5** illustrates a sectional view of portions of the implant apparatus **100** along lines **5-5** of **FIG. 2****,** with the valve prosthesis **102** in the radially-compressed position and received within the outer sheath **136.** It will be appreciated that portions of the implant apparatus **100** are illustrated schematically in **FIGS. 5-14** for illustrative purposes. For example, the valve prosthesis **102** is illustrated somewhat schematically to show a position of the valve prosthesis **102** relative to the inner shaft member **118** and other portions of the implant apparatus **100.** In operation, the outer sheath **136** may be coextensive with the valve prosthesis **102,** such that when the outer sheath **136** is retracted, a distal end (e.g., outflow end **405**) of the valve prosthesis **102** is no longer surrounded by the outer sheath **136,** such that the distal end (e.g., outflow end **405**) can release (e.g., partially deploy, radially expand, etc.). Accordingly, in aspects, the distal end of the valve prosthesis **102** can substantially match a location of the distal end of the outer sheath **136.** The inner shaft member **118** can extend along a shaft axis **501** through the valve prosthesis **102** and through the outer sheath **136.** It will be appreciated that the inner shaft member **118** can be bent, curved, flexed, or otherwise positioned in a non-linear orientation during movement through the patient's vasculature. The inner shaft member **118** can extend between a proximal shaft end **503** and a distal shaft end **505.** The proximal shaft end **503** may be located near the proximal end **106** of the implant apparatus **100,** and the distal shaft end **505** may be located near the distal end **104** of the implant apparatus **100.** The inner shaft member **118** can comprise at least one lumen extending at least partially along a length of the inner shaft member **118** between the proximal shaft end **503** and the distal shaft end **505.** For example, the inner shaft member **118** can comprise a first lumen **507** extending between a first entry opening **509** at the proximal shaft end **503** and a first exit opening **511** at the distal shaft end **505.** In aspects, the first lumen **507** can comprise a first cross-sectional size, for example, a first diameter **513,** that is within a range from about 0.5 millimeters to about 1.5 millimeters. However, based on a cross-sectional size of a guidewire that is received within the first lumen **507,** which may be greater than 1.5 millimeters or less than 0.5 millimeters, the first diameter **513** of the first lumen **507** can be larger or smaller.

In addition, the inner shaft number **118** may comprise a second lumen **515** extending adjacent to the first lumen **507** between a second entry opening **517** at the proximal shaft end **503** and a second exit opening **519** spaced a distance **520** from the distal shaft end **505.** A shaft wall **521** may be positioned between the first lumen **507** and the second lumen **515,** with the shaft wall **521** extending partially or completely along the length of the inner shaft member **118** between the proximal shaft end **503** and the distal shaft end **505.** In this way, the shaft wall **521** can separate the first lumen **507** from the second lumen **515,** such that one guidewire can be maintained in the first lumen **507** and a separate guidewire can be maintained in the second lumen **515.** In aspects, at least a portion of the first lumen **507** may be substantially parallel to at least a portion of the second lumen **515.** The second lumen **515** can comprise a second cross-sectional size, for example, a second diameter **525,** that is within a range from about 0.5 millimeters to about 1.5 millimeters. However, based on a cross-sectional size of a guidewire that is received within the second lumen **515,** which may be greater than 1.5 millimeters or less than 0.5 millimeters, the second diameter **525** of the second lumen **515** can be larger or smaller. In aspects, the inner shaft member **118** can comprise a cross-sectional size (e.g., diameter) that is within a range from about 2 millimeters to about 4 millimeters, or about 2.5 millimeters to about 3.5 millimeters, or about 3 millimeters.

In this way, in some aspects, the first diameter **513** may be substantially equal to the second diameter **525.** The second lumen **515** can comprise an end portion **527** that extends between the second exit opening **519** and the portion of the second lumen **515** that is parallel to the first lumen **507.** In aspects, the end portion **527** can extend along a lumen axis **529** that is angled and non-parallel relative to the shaft axis **501** and angled and non-parallel to the first lumen **507.** The end portion **527** can extend radially outwardly from the second lumen **515.** In this way, the second exit opening **519** can be formed at, or can extend through, a circumferential sidewall **531** of the inner shaft member **118** while the first exit opening **511** can be formed at, or can extend through, an end wall **533** of the inner shaft member **118.** The circumferential sidewall **531** may extend along the shaft axis **501** while the end wall **533** can be perpendicular to, and intersected by, the shaft axis **501.**

The tip **124** can be attached to the distal shaft end **505** and may comprise a tip lumen **537** that is aligned with the first lumen **507.** As used herein, by being aligned with, the first lumen **507** and the tip lumen **537** can be arranged end-to-end such that an axis can extend from the first lumen **507** and into the tip lumen **537** without intersecting a wall or other solid structure. In this way, a guidewire can extend from the first lumen **507** and into the tip lumen **537.** The second exit opening **519** may be spaced apart from the tip **124,** for example, by being spaced the distance **520** from the tip **124.**

The outer sheath **136** can circumferentially surround the valve prosthesis **102** (e.g., when the valve prosthesis **102** is in the radially-compressed position) and the inner shaft member **118.** The outer sheath **136** is movable relative to the valve prosthesis **102** and the inner shaft member **118.** In aspects, the outer sheath **136** is movable relative to the inner shaft member **118** between a first delivery position (e.g., illustrated in **FIGS. 2****,** **5****,** **6****,** and **10**), in which the second exit opening **519** is within a sheath chamber **541** of the outer sheath **136,** and a second delivery position (e.g., illustrated in **FIGS. 3, 4****, 7-9,** and **11-14**), in which the second exit opening **519** is outside of the sheath chamber **541.** The sheath chamber **541** comprises the region that is circumferentially surrounded by the outer sheath **136.** In this way, by being within the sheath chamber **541** in the first delivery position, the second exit opening **519** may be blocked by the outer sheath **136** such that a radial axis perpendicular to the shaft axis **501** can intersect the second exit opening **519** and the outer sheath **136.** Conversely, by being outside of the sheath chamber **541** in the second delivery position, the second exit opening **519** is not blocked by the outer sheath **136** such that a radial axis perpendicular to the shaft axis **501** can intersect the second exit opening **519** but may not intersect the outer sheath **136.**

The guide member **114** can be attached to the proximal shaft end **503** of the inner shaft member **118.** In aspects, the guide member **114** can comprise a plurality of passageways that may be aligned with the first lumen **507** and the second lumen **515.** For example, the guide member **114** can comprise a first passageway **545** that is aligned with the first entry opening **509** of the first lumen **507,** with the first passageway **545** extending along a first guide axis **547** that can extend through the first entry opening **509.** The guide member **114** can comprise a second passageway **551** that is aligned with the second entry opening **517** of the second lumen **515,** with the second passageway **551** extending along a second guide axis **553** that can extend through the second entry opening **517.** In aspects, the first guide axis **547** may be non-parallel relative to the second guide axis **553.** For example, an angle **555** between the first guide axis **547** and the second guide axis **553** may be within a range from about 20 degrees to about 60 degrees. The guide member **114** can allow for a physician to insert one guidewire through the first passageway **545** and the first lumen **507,** and insert a separate guidewire through the second passageway **551** and the second lumen **515.** Further, the physician can hold the guide member **114** and control axial movement of the guidewires.

**FIG. 6** illustrates the insertion of a first guidewire **601** within the inner shaft member **118.** In this way, methods of implanting the heart valve prosthesis **102** can comprise positioning the first guidewire **601** within the first lumen **507** of the inner shaft member **118,** with the first lumen **507** extending between the proximal shaft end **503** and the distal shaft end **505.** For example, the first guidewire **601** can be positioned to extend through the first passageway **545** of the guide member **114,** the first entry opening **509,** the first lumen **507,** the first exit opening **511,** and the tip lumen **537.** In this way, the first guidewire **601** can extend at least partially along the first guide axis **547.** The first guidewire **601** can exit the implant apparatus **100** through the tip lumen **537** at the distal end **104.** The first guidewire **601** comprises a cross-sectional size that is less than the first diameter **513** of the first lumen **507,** such that the first guidewire **601** can move relative to the inner shaft member **118** and, likewise, the inner shaft member **118** can move relative to the first guidewire **601.**

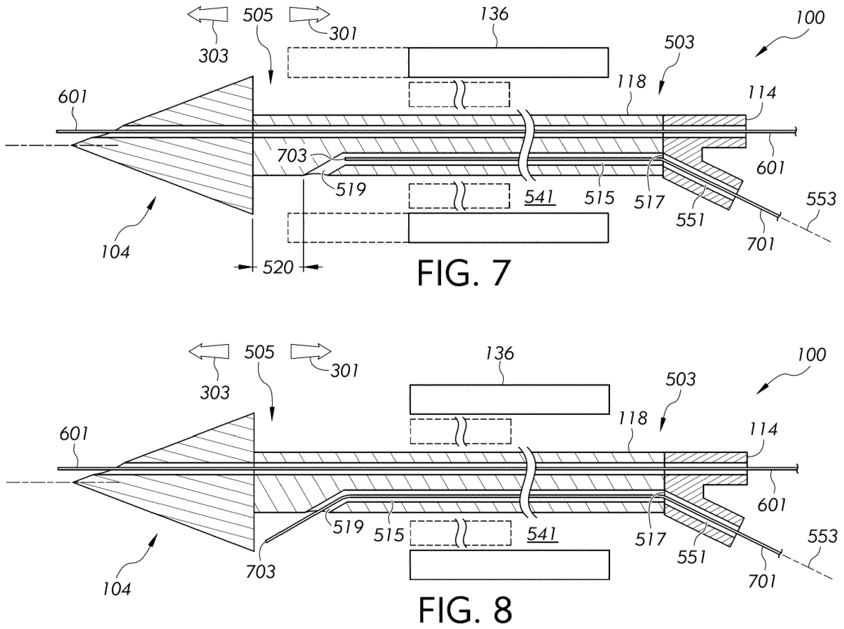

**FIG. 7** illustrates the insertion of a second guidewire **701** within the inner shaft member **118.** In this way, methods can comprise positioning the second guidewire **701** within the second lumen **515,** with the second lumen **515** extending between the proximal shaft end **503** and the second exit opening **519** that is spaced the distance **520** from the distal shaft end **505.** For example, the second guidewire **701** can extend through the second passageway **551,** the second entry opening **517,** and the second lumen **515.** In this way, the portion of the second guidewire **701** extending through the second passageway **551** can extend along the second guide axis **553.** In aspects, a second guidewire end **703** of the second guidewire **701** can remain within the second lumen **515** during movement of the implant apparatus **100.** For example, methods can comprise moving (e.g., in the distal direction **303**) the transcatheter heart valve implant apparatus **100** comprising the inner shaft member **118** and the valve prosthesis **102** along the first guidewire **601** toward a treatment site within a body lumen. During the movement of the transcatheter heart valve implant apparatus **100,** the outer sheath **136** is in the first delivery position (e.g., illustrated in **FIGS. 5, 6****,** and with dashed lines in **FIG. 7**) and can circumferentially surround the valve prosthesis **102** and the inner shaft member **118,** with the second exit opening **519** located within the sheath chamber **541** of the outer sheath **136.**

In aspects, upon reaching the treatment site, the outer sheath **136** can be moved in the proximal direction **301** and/or the inner shaft member **118** can be moved in the distal direction **303** such that the outer sheath **136** can be moved from the first delivery position to the second delivery position (e.g., illustrated with solid lines). In the second delivery position, the second exit opening **519** is not located within the sheath chamber **541.** In this way, methods can comprise, prior to guiding the second guidewire **701** through the second exit opening **519,** moving one or more of the outer sheath **136** relative to the inner shaft member **118** or the inner shaft member **118** relative to the outer sheath **136** such that the second exit opening **519** may be outside the sheath chamber **541.**

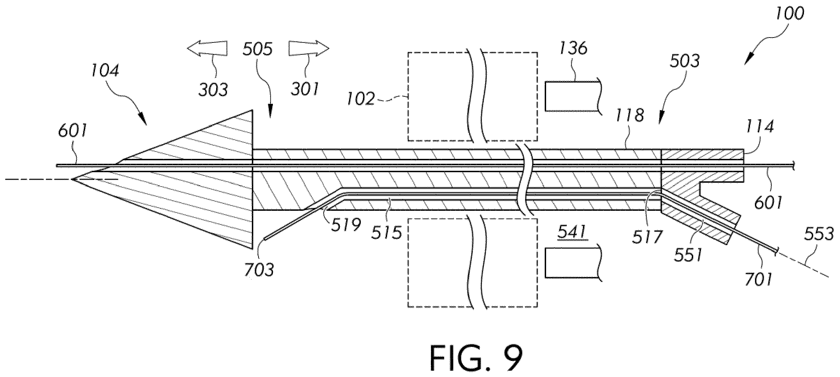

**FIG. 8** illustrates the second guidewire **701** moving in the distal direction **303** relative to the inner shaft member **118** and passing through the second exit opening **519.** The second guidewire **701** can be guided through the second exit opening **519** such that the second guidewire end **703** can be outside of the second lumen **515.** To move the second guidewire **701,** a physician can apply an axial force to the second guidewire **701** at the guide member **114** in the distal direction **303.** This axial force can cause the second guidewire **701** to move through the second lumen **515** and exit the second lumen **515** through the second exit opening **519.** Referring to **FIG. 9****,** after the second guidewire **701** has exited the second exit opening **519,** the valve prosthesis **102** can move from the radially-compressed position (e.g., illustrated in **FIG. 8**) to the radially-expanded position illustrated in **FIG. 9****.** For example, the outer sheath **136** can be retracted, for example, by being moved in the proximal direction **301,** such that the valve prosthesis **102** is not positioned in the sheath chamber **541.** As described above relative to **FIGS. 3-4****,** the coil **132** can be rotated to release the inflow end **403** of the valve prosthesis **102,** thus allowing the valve prosthesis **102** to expand to the radially-expanded position.

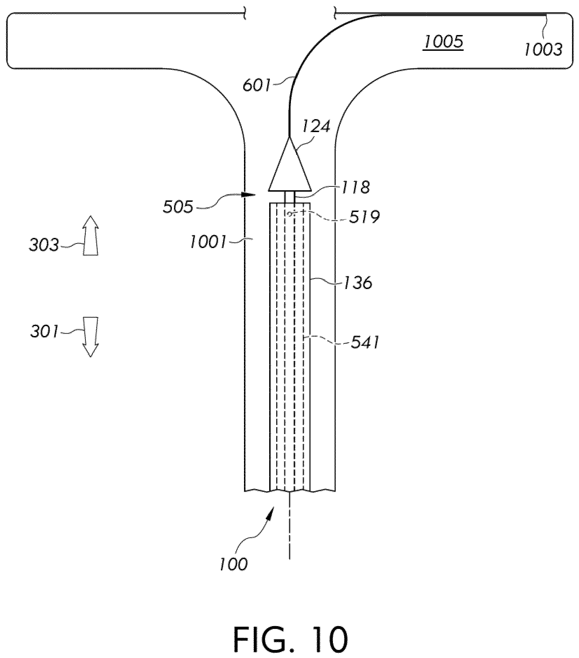

**FIGS. 10-14** schematically illustrate a side view of the transcatheter heart valve implant apparatus **100** at a treatment site **1001.** For example, **FIG. 10** is a side view of the transcatheter heart valve implant apparatus **100** in a position similar to **FIG. 6****.** In this way, a first guidewire end **1003** of the first guidewire **601** may be positioned at a first anchoring location **1005.** In aspects, the first anchoring location **1005** can comprise a first pulmonary artery branch, such as the right pulmonary artery. Accordingly, methods can comprise guiding the first guidewire **601** through the first exit opening **511** (e.g., illustrated in **FIG. 5**) at the distal shaft end **505** such that the first guidewire end **1003** of the first guidewire **601** can be positioned in a first anchoring location, such as a first pulmonary artery branch. The implant apparatus **100** can move relative to the first guidewire **601,** for example, in the distal direction **303,** toward the treatment site **1001.** In aspects, during the movement of the implant apparatus **100** in the distal direction **303,** the second guidewire end **703** (e.g., illustrated in **FIG. 7**) can be located within the second lumen **515.**

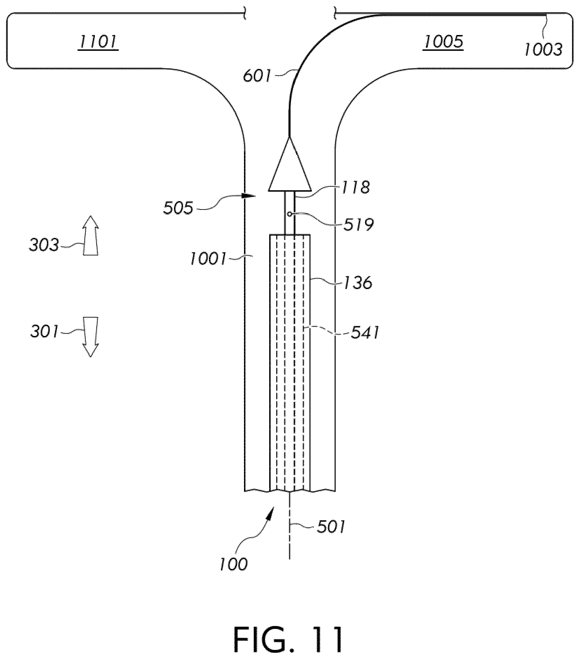

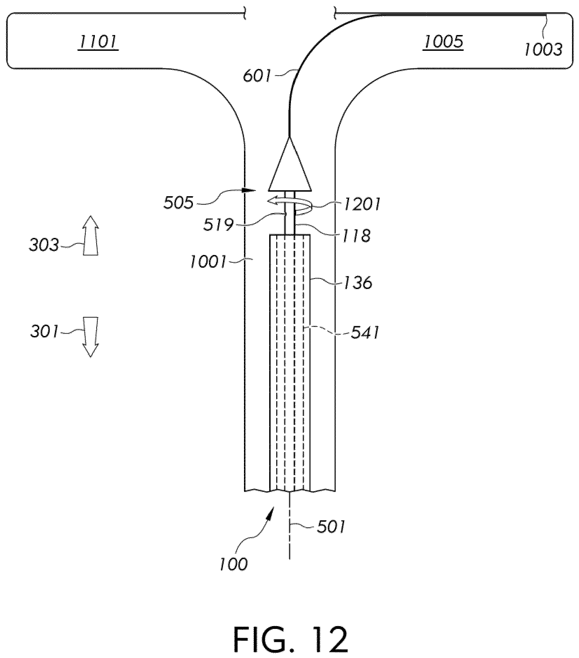

**FIG. 11** illustrates a position of the transcatheter heart valve implant apparatus **100** substantially similar to **FIG. 7****.** For example, the outer sheath **136** can be moved relative to the inner shaft member **118** and/or the inner shaft member **118** can be moved relative to the outer sheath **136.** In this way, the second exit opening **519** may be outside of the sheath chamber **541** and no longer covered or blocked by the outer sheath **136.** Referring to **FIGS. 11-12****,** in aspects, prior to guiding the second guidewire **701** through the second exit opening **519,** methods can comprise rotating the inner shaft member **118** relative to the outer sheath **136** such that the second exit opening **519** may be oriented to face a second anchoring location **1101.** The inner shaft member **118** can be rotated about the shaft axis **501.** The second anchoring location **1101** can comprise, for example, a second anchoring location, such as, for example, a second pulmonary artery branch (e.g., the left pulmonary artery). For example, the inner shaft member **118** can be rotatable relative to the outer sheath **136** about the shaft axis **501** such that in a first rotational position (e.g., as illustrated in **FIG. 11**), the second exit opening **519** faces a first location of the outer sheath **136** and may not face the second anchoring location **1101.** The inner shaft member **118** can be rotated (e.g., rotation illustrated schematically with arrowhead **1201** about the shaft axis **501** in **FIG. 12**) from the first rotational position of **FIG. 11** to a second rotational position of **FIG. 12****,** in which the second exit opening **519** faces a second location of the outer sheath **136** and faces the second anchoring location **1101.** In this way, the rotation **1201** can occur while the second exit opening **519** is outside of the sheath chamber **541.** The inner shaft member **118** can be rotated in a clockwise direction or a counter-clockwise direction, with the end result being that the second exit opening **519** faces the second anchoring location **1101.** In aspects, by facing the second anchoring location **1101,** an axis can extend from the second exit opening **519** and into the second anchoring location **1101** without intersecting any structures, such as native anatomical structures of the patient and/or components of the implant apparatus **100.** Accordingly, in this way, when the inner shaft member **118** is in the second rotational position of **FIG. 12****,** an unobstructed path may exist between the second exit opening **519** and the second anchoring location **1101.**

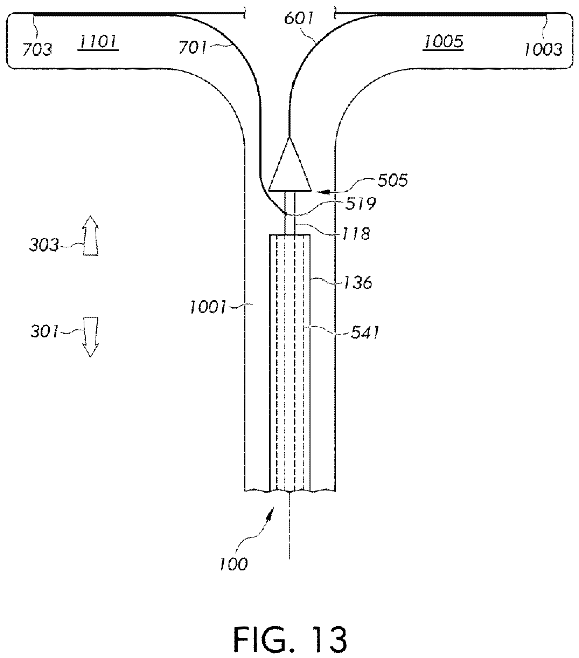

**FIG. 13** illustrates a position of the transcatheter heart valve implant apparatus **100** substantially similar to **FIG. 8****.** For example, the second guidewire **701** can be guided through the second exit opening **519** such that the second guidewire end **703** can be positioned in the second anchoring location **1101,** for example, the second pulmonary artery branch. By guiding the second guidewire **701** through the second exit opening **519,** the second guidewire **701** can be moved in the distal direction **303** such that the second guidewire end **703** moves toward the second anchoring location **1101.** The second guidewire **701** can continue to be moved in the distal direction **303** at least until the second guidewire end **703** is located in the second anchoring location **1101,** for example, by contacting a wall of the second pulmonary artery branch. As described above, the first guidewire **601** and the second guidewire **701** can be moved in the distal direction **303** due to a force being applied to the guidewires **601, 701** at the guide member **114** (e.g., illustrated in **FIGS. 5-9**).

In this way, the first guidewire **601** and the second guidewire **701** may be simultaneously deployed prior to the deployment of the valve prosthesis **102,** with the first guidewire **601** in the first anchoring location **1005** and the second guidewire **701** in the second anchoring location **1101.** In aspects, the first guidewire **601,** for example, the first guidewire end **1003,** can be in contact with a wall of the first anchoring location **1005.** Likewise, the second guidewire **701,** for example, the second guidewire end **703,** can be in contact with a wall of the second anchoring location **1101.** In aspects, the first guidewire **601** and the second guidewire **701** can be spaced apart, for example, within a range from about 135 degrees to about 225 degrees, or from about 160 degrees to about 200 degrees. Together, the first guidewire **601** and the second guidewire **701** can function to provide opposing forces to the inner shaft member **118** and limit unintended movement of the implant apparatus **100** during deployment of the valve prosthesis **102.** In aspects, the anchoring locations **1005, 1101** for the first guidewire **601** and the second guidewire **701** can be switched. For example, the first guidewire **601** can be positioned in the second anchoring location **1101** (e.g., the left pulmonary artery), and the second guidewire **701** can be positioned in the first anchoring location **1005** (e.g., the right pulmonary artery). Despite the guidewires **601, 701** being capable of positioning in either of the anchoring locations **1005, 1101,** the guidewires **601, 701** can be simultaneously deployed. As used herein, the term simultaneous (e.g., simultaneous deployment, simultaneous positioning, etc.) can refer to the first guidewire **601** and the second guidewire **701** both extending, at the same time, to an exterior of the inner shaft member **118** and extending into differing anchoring locations. In this way, at a given moment, the first guidewire **601** may extend into the first anchoring location **1005** at the same time as the second guidewire **701** extending into the second anchoring location **1101.**

**FIG. 14** illustrates the deployment of the valve prosthesis **102** in a similar manner as described relative to **FIG. 9****.** As the valve prosthesis **102** is deployed, the first guidewire **601** and the second guidewire **701** can anchor the inner shaft member **118** to ensure that the valve prosthesis **102** is deployed at a desired location. For example, during deployment, the valve prosthesis **102** may be in proximity to a wall **1401** of the treatment site **1001.** As the valve prosthesis **102** radially expands from the radially-contracted position (e.g., illustrated in **FIG. 13**) to the radially-expanded position (e.g., illustrated in **FIG. 14**), the valve prosthesis **102** may contact the wall **1401.** Contact between the valve prosthesis **102** and the wall **1401** may generate a force **1403** (e.g., illustrated schematically with arrowhead) acting upon the valve prosthesis **102** in a direction away from the wall **1401.** In aspects, this force **1403** can tend to move the valve prosthesis **102** and cause an inaccurate positioning of the valve prosthesis **102** within the treatment site **1001.**

The simultaneous anchoring and positioning of the first guidewire **601** in the first anchoring location **1005** and the second guidewire **701** in the second anchoring location **1101** can counteract the force **1403** and limit unintended movement of the valve prosthesis **102.** For example, the simultaneous anchoring of the first guidewire **601** and the second guidewire **701** can provide a balanced reaction force on the inner shaft member **118,** such that the inner shaft member **118** can remain centered as the valve prosthesis **102** is deployed and even if the valve prosthesis **102** contacts the wall **1401.** That is, in aspects, the first anchoring location **1005** and the second anchoring location **1101** are angularly spaced apart, with the guidewires **601, 701** in contact with walls of the anchoring locations **1005, 1101.** The guidewires **601, 701** can therefore assist in resisting movement of the inner shaft member **118** and the valve prosthesis **102** despite the force **1403** possibly acting upon the valve prosthesis **102.** Accordingly, the valve prosthesis **102** can be positioned at a desired location at the treatment site **1001** with a reduced likelihood of misplacement. Following the deployment of the valve prosthesis **102,** the guidewires **601, 701** can be retracted in the proximal direction **301** and the implant apparatus **100** can be removed from the vasculature of the patient. The implant apparatus **100** described herein can provide several benefits, such as, for example, more accurate positioning of the valve prosthesis **102.** In addition, the processes and structures related to the deployment of the valve prosthesis **102** need not be changed. That is, the holding catheter **128,** the coil **132,** etc. can function in a similar manner as existing techniques.

It should be understood that while various aspects have been described in detail relative to certain illustrative and specific examples thereof, the present disclosure should not be considered limited to such, as numerous modifications and combinations of the disclosed features are possible without departing from the scope of the following claims.

Further disclosed herein is a transcatheter heart valve implant apparatus including a valve prosthesis extending along a longitudinal axis. An inner shaft member extends along a shaft axis through the valve prosthesis between a proximal shaft end and a distal shaft end. The inner shaft member includes a first lumen extending between the proximal shaft end and a first exit opening at the distal shaft end. The inner shaft member includes a second lumen extending between the proximal shaft end and a second exit opening spaced from the distal shaft end. A first guidewire extends through the first lumen and the first exit opening. A first end of the first guidewire is at a first anchoring location. A second guidewire extends through the second lumen and the second exit opening. A second end of the second guidewire is at a second anchoring location. Methods of implanting a valve prosthesis are provided.

## Claims

1. A transcatheter heart valve implant apparatus (100) comprising:
an annular valve prosthesis (102) extending along a longitudinal axis (401) between an inflow end (403) of the valve prosthesis and an outflow end (405) of the valve prosthesis;
an inner shaft member (118) extending along a shaft axis (501) through the valve prosthesis between a proximal shaft end (503) and a distal shaft end (505), the inner shaft member comprising:
a first lumen (507) extending between the proximal shaft end and a first exit opening (511) at the distal shaft end; and
a second lumen (515) extending adjacent to the first lumen between the proximal shaft end and a second exit opening (519) spaced a distance from the distal shaft end, a shaft wall (521) positioned between the first lumen and the second lumen;
a first guidewire (601) extending through the first lumen and the first exit opening, wherein a first end of the first guidewire is configured to be positioned at a first anchoring location (1005); and
a second guidewire (701) extending through the second lumen and the second exit opening, wherein a second end of the second guidewire is configured to be positioned at a second anchoring location (1101) simultaneously with the first guidewire positioned at the first anchoring location.

2. The transcatheter heart valve implant apparatus of claim 1, further comprising a tip (124) attached to the distal shaft end, the tip comprising a tip lumen (537) that is aligned with the first lumen such that the first guidewire extends through the tip lumen.

3. The transcatheter heart valve implant apparatus of claim 2, wherein the second exit opening is spaced apart from the tip.

4. The transcatheter heart valve implant apparatus of any preceding claim, wherein the first anchoring location comprises a first pulmonary artery branch and the second anchoring location comprises a second pulmonary artery branch.

5. The transcatheter heart valve implant apparatus of any preceding claim, further comprising an outer sheath (122) circumferentially surrounding the valve prosthesis and the inner shaft member, the outer sheath movable relative to the inner shaft member such that in a first delivery position, the second exit opening is within a sheath chamber (541) of the outer sheath, and in a second delivery position, the second exit opening is outside of the sheath chamber.

6. The transcatheter heart valve implant apparatus of claim 5, wherein the inner shaft member is configured to be rotatable relative to the outer sheath such that in a first rotational position, the second exit opening faces a first location of the outer sheath, and in a second rotational position, the second exit opening faces a second location of the outer sheath.

7. The transcatheter heart valve implant apparatus of any preceding claim, wherein the first lumen comprises a first diameter (513) that is within a range from about 0.5 millimeters to about 1.5 millimeters, and the second lumen comprises a second diameter (525) that is within a range from about 0.5 millimeters to about 1.5 millimeters.

8. The transcatheter heart valve implant apparatus of any preceding claim, wherein the first lumen is substantially parallel to the second lumen.

9. The transcatheter heart valve implant apparatus of any preceding claim,
wherein the first lumen extends between a first entry opening (509) at the proximal shaft end and the first exit opening at the distal shaft end;
wherein the second lumen extends between a second entry opening (517) at the proximal shaft end and the second exit opening;
wherein the first guidewire extends through the first entry opening, , wherein the first end of the first guidewire is configured to be positioned in a first pulmonary artery branch; and
wherein the second guidewire extends through the second entry opening, wherein the second end of the second guidewire is configured to be positioned in a second pulmonary artery branch simultaneously with the first guidewire positioned in the first pulmonary artery branch.

10. The transcatheter heart valve implant apparatus of claim 9, further comprising a guide member (114) attached to the proximal shaft end of the inner shaft member.

11. The transcatheter heart valve implant apparatus of claim 10, wherein the guide member comprises:
a first passageway (545) that is aligned with the first entry opening of the first lumen such that the first guidewire extends through the first passageway, the first entry opening, and the first lumen, the first passageway extending along a first guide axis (547); and
a second passageway (551) that is aligned with the second entry opening of the second lumen such that the second guidewire extends through the second passageway, the second entry opening, and the second lumen, the second passageway extending along a second guide axis (553).

12. The transcatheter heart valve implant apparatus of claim 11, wherein an angle (555) between the first guide axis and the second guide axis is within a range from about 20 degrees to about 60 degrees.

## Patentansprüche

1. Transkatheter-Herzklappenimplantatvorrichtung (100), umfassend:
eine ringförmige Klappenprothese (102), die sich entlang einer Längsachse (401) zwischen einem Einströmende (403) der Klappenprothese und einem Ausströmende (405) der Klappenprothese erstreckt;
ein inneres Schaftelement (118), das sich entlang einer Schaftachse (501) durch die Klappenprothese zwischen einem proximalen Schaftende (503) und einem distalen Schaftende (505) erstreckt, wobei das innere Schaftelement umfasst:
ein erstes Lumen (507), das sich zwischen dem proximalen Schaftende und einer ersten Austrittsöffnung (511) an dem distalen Schaftende erstreckt; und
ein zweites Lumen (515), das sich angrenzend an das erste Lumen zwischen dem proximalen Schaftende und einer zweiten Austrittsöffnung (519) erstreckt, die in einem Abstand von dem distalen Schaftende angeordnet ist, wobei eine Schaftwand (521) zwischen dem ersten Lumen und dem zweiten Lumen positioniert ist;
einen ersten Führungsdraht (601), der sich durch das erste Lumen und die erste Austrittsöffnung erstreckt, wobei ein erstes Ende des ersten Führungsdrahts konfiguriert ist, um an einer ersten Verankerungsstelle (1005) positioniert zu werden; und
einen zweiten Führungsdraht (701), der sich durch das zweite Lumen und die zweite Austrittsöffnung erstreckt, wobei ein zweites Ende des zweiten Führungsdrahts konfiguriert ist, um an einer zweiten Verankerungsstelle (1101) gleichzeitig mit dem an der ersten Verankerungsstelle positionierten ersten Führungsdraht positioniert zu werden.

2. Transkatheter-Herzklappenimplantatvorrichtung nach Anspruch 1, ferner umfassend eine Spitze (124), die an dem distalen Schaftende angebracht ist, wobei die Spitze ein Spitzenlumen (537) umfasst, das mit dem ersten Lumen ausgerichtet ist, sodass sich der erste Führungsdraht durch das Spitzenlumen erstreckt.

3. Transkatheter-Herzklappenimplantatvorrichtung nach Anspruch 2, wobei die zweite Austrittsöffnung von der Spitze beabstandet ist.

4. Transkatheter-Herzklappenimplantatvorrichtung nach einem der vorstehenden Ansprüche, wobei die erste Verankerungsstelle einen ersten Pulmonalarterienzweig umfasst und die zweite Verankerungsstelle einen zweiten Pulmonalarterienzweig umfasst.

5. Transkatheter-Herzklappenimplantatvorrichtung nach einem der vorstehenden Ansprüche, ferner umfassend eine äußere Hülle (122), die die Klappenprothese und das innere Schaftelement umlaufend umgibt, wobei die äußere Hülle relativ zu dem inneren Schaftelement beweglich ist, sodass sich in einer ersten Zuführposition die zweite Austrittsöffnung innerhalb einer Hüllenkammer (541) der äußeren Hülle befindet und sich in einer zweiten Zuführposition die zweite Austrittsöffnung außerhalb der Hüllenkammer befindet.

6. Transkatheter-Herzklappenimplantatvorrichtung nach Anspruch 5, wobei das innere Schaftelement so konfiguriert ist, dass es relativ zu der äußeren Hülle drehbar ist, sodass in einer ersten Drehposition die zweite Austrittsöffnung einer ersten Stelle der äußeren Hülle zugewandt ist und in einer zweiten Drehposition die zweite Austrittsöffnung einer zweiten Stelle der äußeren Hülle zugewandt ist.

7. Transkatheter-Herzklappenimplantatvorrichtung nach einem der vorstehenden Ansprüche, wobei das erste Lumen einen ersten Durchmesser (513) umfasst, der in einem Bereich von etwa 0,5 Millimetern bis etwa 1,5 Millimetern liegt, und das zweite Lumen einen zweiten Durchmesser (525) umfasst, der in einem Bereich von etwa 0,5 Millimetern bis etwa 1,5 Millimetern liegt.

8. Transkatheter-Herzklappenimplantatvorrichtung nach einem der vorstehenden Ansprüche, wobei das erste Lumen im Wesentlichen parallel zu dem zweiten Lumen ist.

9. Transkatheter-Herzklappenimplantatvorrichtung nach einem der vorstehenden Ansprüche,
wobei sich das erste Lumen zwischen einer ersten Eintrittsöffnung (509) an dem proximalen Schaftende und der ersten Austrittsöffnung an dem distalen Schaftende erstreckt;
wobei sich das zweite Lumen zwischen einer zweiten Eintrittsöffnung (517) an dem proximalen Schaftende und der zweiten Austrittsöffnung erstreckt;
wobei sich der erste Führungsdraht durch die erste Eintrittsöffnung erstreckt, wobei das erste Ende des ersten Führungsdrahts konfiguriert ist, um in einem ersten Pulmonalarterienzweig positioniert zu werden; und
wobei sich der zweite Führungsdraht durch die zweite Eintrittsöffnung erstreckt, wobei das zweite Ende des zweiten Führungsdrahts konfiguriert ist, um in einem zweiten Pulmonalarterienzweig gleichzeitig mit dem in dem ersten Pulmonalarterienzweig positionierten ersten Führungsdraht positioniert zu werden.

10. Transkatheter-Herzklappenimplantatvorrichtung nach Anspruch 9, ferner umfassend ein Führungselement (114), das an dem proximalen Schaftende des inneren Schaftelements angebracht ist.

11. Transkatheter-Herzklappenimplantatvorrichtung nach Anspruch 10, wobei das Führungselement umfasst:
einen ersten Durchgang (545), der mit der ersten Eintrittsöffnung des ersten Lumens ausgerichtet ist, sodass sich der erste Führungsdraht durch den ersten Durchgang, die erste Eintrittsöffnung und das erste Lumen erstreckt, wobei sich der erste Durchgang entlang einer ersten Führungsachse (547) erstreckt; und
einen zweiten Durchgang (551), der mit der zweiten Eintrittsöffnung des zweiten Lumens ausgerichtet ist, sodass sich der zweite Führungsdraht durch den zweiten Durchgang, die zweite Eintrittsöffnung und das zweite Lumen erstreckt, wobei sich der zweite Durchgang entlang einer zweiten Führungsachse (553) erstreckt.

12. Transkatheter-Herzklappenimplantatvorrichtung nach Anspruch 11, wobei ein Winkel (555) zwischen der ersten Führungsachse und der zweiten Führungsachse in einem Bereich von etwa 20 Grad bis etwa 60 Grad liegt.

## Revendications

1. Appareil d'implant valvulaire cardiaque transcathéter (100) comprenant :
une prothèse valvulaire annulaire (102) s'étendant le long d'un axe longitudinal (401) entre une extrémité de débit entrant (403) de la prothèse valvulaire et une extrémité de débit sortant (405) de la prothèse valvulaire ;
un élément de tige interne (118) s'étendant le long d'un axe de tige (501) à travers la prothèse valvulaire entre une extrémité de tige proximale (503) et une extrémité de tige distale (505), l'élément de tige interne comprenant :
une première lumière (507) s'étendant entre l'extrémité de tige proximale et une première ouverture de sortie (511) au niveau de l'extrémité de tige distale ; et
une seconde lumière (515) s'étendant adjacente à la première lumière entre l'extrémité de tige proximale et une seconde ouverture de sortie (519) espacée d'une certaine distance de l'extrémité de tige distale, une paroi de tige (521) positionnée entre la première lumière et la seconde lumière ;
un premier fil-guide (601) s'étendant à travers la première lumière et la première ouverture de sortie, dans lequel une première extrémité du premier fil-guide est conçue pour être positionnée au niveau d'un premier emplacement d'ancrage (1005) ; et
un second fil-guide (701) s'étendant à travers la seconde lumière et la seconde ouverture de sortie, dans lequel une seconde extrémité du second fil-guide est conçue pour être positionnée au niveau d'un second emplacement d'ancrage (1101) simultanément avec le premier fil-guide positionné au niveau du premier emplacement d'ancrage.

2. Appareil d'implant valvulaire cardiaque transcathéter selon la revendication 1, comprenant en outre une pointe (124) fixée à l'extrémité de tige distale, la pointe comprenant une lumière de pointe (537) qui est alignée avec la première lumière de telle sorte que le premier fil-guide s'étend à travers la lumière de pointe.

3. Appareil d'implant valvulaire cardiaque transcathéter selon la revendication 2, dans lequel la seconde ouverture de sortie est espacée de la pointe.

4. Appareil d'implant valvulaire cardiaque transcathéter selon l'une quelconque revendication précédente, dans lequel le premier emplacement d'ancrage comprend une première branche d'artère pulmonaire et le second emplacement d'ancrage comprend une seconde branche d'artère pulmonaire.

5. Appareil d'implant valvulaire cardiaque transcathéter selon l'une quelconque revendication précédente, comprenant en outre une gaine externe (122) entourant circonférentiellement la prothèse valvulaire et l'élément de tige interne, la gaine externe pouvant être déplacée par rapport à l'élément de tige interne de telle sorte que dans une première position de délivrance, la seconde ouverture de sortie est au sein d'une chambre de gaine (541) de la gaine externe, et dans une seconde position de délivrance, la seconde ouverture de sortie est à l'extérieur de la chambre de gaine.

6. Appareil d'implant valvulaire cardiaque transcathéter selon la revendication 5, dans lequel l'élément de tige interne est conçu pour être rotatif par rapport à la gaine externe de telle sorte que dans une première position de rotation, la seconde ouverture de sortie fait face à un premier emplacement de la gaine externe, et dans une seconde position de rotation, la seconde ouverture de sortie fait face à un second emplacement de la gaine externe.

7. Appareil d'implant valvulaire cardiaque transcathéter selon l'une quelconque revendication précédente, dans lequel la première lumière comprend un premier diamètre (513) qui est au sein d'une plage d'environ 0,5 millimètre à environ 1,5 millimètre, et la seconde lumière comprend un second diamètre (525) qui est au sein d'une plage d'environ 0,5 millimètre à environ 1,5 millimètre.

8. Appareil d'implant valvulaire cardiaque transcathéter selon l'une quelconque revendication précédente, dans lequel la première lumière est sensiblement parallèle à la seconde lumière.

9. Appareil d'implant valvulaire cardiaque transcathéter selon l'une quelconque revendication précédente,
dans lequel la première lumière s'étend entre une première ouverture d'entrée (509) au niveau de l'extrémité de tige proximale et la première ouverture de sortie au niveau de l'extrémité de tige distale ;
dans lequel la seconde lumière s'étend entre une seconde ouverture d'entrée (517) au niveau de l'extrémité de tige proximale et la seconde ouverture de sortie ;
dans lequel le premier fil-guide s'étend à travers la première ouverture d'entrée, dans lequel la première extrémité du premier fil-guide est conçue pour être positionnée dans une première branche d'artère pulmonaire ; et
dans lequel le second fil-guide s'étend à travers la seconde ouverture d'entrée, dans lequel la seconde extrémité du second fil-guide est conçue pour être positionnée dans une seconde branche d'artère pulmonaire simultanément avec le premier fil-guide positionné dans la première branche d'artère pulmonaire.

10. Appareil d'implant valvulaire cardiaque transcathéter selon la revendication 9, comprenant en outre un élément de guidage (114) fixé à l'extrémité de tige proximale de l'élément de tige interne.

11. Appareil d'implant valvulaire cardiaque transcathéter selon la revendication 10, dans lequel l'élément de guidage comprend :
une première voie de passage (545) qui est alignée avec la première ouverture d'entrée de la première lumière de telle sorte que le premier fil-guide s'étend à travers la première voie de passage, la première ouverture d'entrée, et la première lumière, la première voie de passage s'étendant le long d'un premier axe de guidage (547) ; et
une seconde voie de passage (551) qui est alignée avec la seconde ouverture d'entrée de la seconde lumière de telle sorte que le second fil-guide s'étend à travers la seconde voie de passage, la seconde ouverture d'entrée, et la seconde lumière, la seconde voie de passage s'étendant le long d'un second axe de guidage (553).

12. Appareil d'implant valvulaire cardiaque transcathéter selon la revendication 11, dans lequel un angle (555) entre le premier axe de guidage et le second axe de guidage est au sein d'une plage d'environ 20 degrés à environ 60 degrés.
